# EUROPEAN PATENT SPECIFICATION

(11) **EP 2 405 021 B1**
(45) Date of publication and mention of the grant of the patent: **13.11.2013**
(21) Application number: 11172734.3
(22) Date of filing: 05.07.2011
(51) Int. Cl.: C12Q 1/68, C07K 1/26, G01N 27/26

(54) **Nucleic acid concentration recovery cartridge, nucleic acid concentration recovery method, and fabrication process of cartridge**
Nukleinsäurekonzentrationsapparat, Nukleinsäurekonzentrationsverfahren und Verfahren zur Herstellung des Apparats
Cartouche de récupération de concentration d'acide nucléique, procédé de récupération de concentration d'acide nucléique et procédé de fabrication de cartouche

(30) Priority: 07.07.2010 JP 2010154692; 31.01.2011 JP 2011017757
(43) Date of publication of application: 11.01.2012
(73) Proprietor: Sony Corporation, Tokyo 108-0075 (JP)
(72) Inventor: Nakamura, Tomohiko, Minato-ku, Tokyo 108-0075 (JP)
(74) Representative: Horner, David Richard

(56) References cited:
- WO-A2-02/42500
- BROEMELING ET AL: "An Instrument for Automated Purification of Nucleic Acids from Contaminated Forensic Samples", JOURNAL OF THE ASSOCIATION FOR LABORATORY AUTOMATION, ELSEVIER, vol. 13, no. 1, 4 January 2008 (2008-01-04), pages 40-48, XP022409935, ISSN: 1535-5535, DOI: 10.1016/J.JALA.2007.10.008
- ALLINGTON W B ET AL: "Electrophoretic concentration of macromolecules", ANALYTICAL BIOCHEMISTRY, ACADEMIC PRESS INC, NEW YORK, vol. 85, no. 1, 1 March 1978 (1978-03-01), pages 188-196, XP024818072, ISSN: 0003-2697, DOI: 10.1016/0003-2697(78)90289-0 [retrieved on 1978-03-01]
- ATMEH ET AL: "Concentration of dilute solutions of macromolecules by electrophoresis", ANALYTICAL BIOCHEMISTRY, ACADEMIC PRESS INC, NEW YORK, vol. 182, no. 2, 1 November 1989 (1989-11-01), pages 315-318, XP024820624, ISSN: 0003-2697, DOI: 10.1016/0003-2697(89)90601-5 [retrieved on 1989-11-01]
- WACHSLICHT H ET AL: "A simple device for protein concentration by steady-state stacking", ANALYTICAL BIOCHEMISTRY, ACADEMIC PRESS INC, NEW YORK, vol. 84, no. 2, 1 February 1978 (1978-02-01), pages 533-538, XP024825001, ISSN: 0003-2697, DOI: 10.1016/0003-2697(78)90072-6 [retrieved on 1978-02-01]
- I POSNER: "The concentration of macromolecules by electrophoresis-sedimentation", ANALYTICAL BIOCHEMISTRY, vol. 70, no. 1, 1 January 1976 (1976-01-01), pages 187-194, XP009154154, ISSN: 0003-2697, DOI: 10.1016/S0003-2697(76)80061-9

## Description

### BACKGROUND

The present technology relates to a nucleic acid concentration recovery cartridge, a nucleic acid concentration recovery method, and a fabrication process of the cartridge.

Nucleic acid amplification reactions such as the PCR (Polymerase Chain Reaction) method and the LAMP (Loop-Mediated Isothermal Amplification) method have found utility in various fields of biotechnology. In the medical field, for example, diagnoses are performed based on DNA (Deoxyribonucleic acid) or RNA (Ribonucleic acid) base sequences, and in the agricultural field, DNA identification is applied for the determination or the like of genetically-modified crops.

According to a nucleic acid amplification reaction, a nucleic acid in a trace sample can be amplified and detected with high efficiency. However, the content of the nucleic acid may be lower than its lower detection limit when the nucleic acid contained in the sample is in an extremely trace amount. Moreover, when the concentration of the nucleic acid in the sample is extremely low, it may be impossible to detect the nucleic acid as the nucleic acid to be amplified is not contained in a sample of a volume that can be introduced to a reaction site. In these instances, it is effective to introduce the sample to a reaction site after the nucleic acid in the sample is concentrated beforehand.

In the past, the method that uses phenol, chloroform or ethanol, the method making use of a column, filter or the like that adsorbs a nucleic acid, the method that uses magnetic silica beads, and the like were known as nucleic acid concentration methods. For example, Japanese Patent Laid-open No. 2005-080555 discloses a concentration method of a nucleic acid, which uses a porous carrier having nucleic acid adsorption capacity. Further, a method that concentrates a nucleic acid by capillary electrophoresis is disclosed in "On-line sample preconcentration in capillary electrophoresis: Fundamentals and applications," Journal of Chromatography A., Vol. 1184, Pages 504 to 541 (2008).

The related method that makes use of phenol, chloroform or ethanol has to use the noxious organic solvent, and is laborious for centrifugal operation or the like. The method making use of a column, filter or the like that can adsorb a nucleic acid is accompanied by a problem from the standpoint of the simplicity and convenience of operation because the column, filter or the like is prone to clogging.

It is, therefore, desirable to provide a nucleic acid concentration recovery cartridge that is simple and convenient in operation and can concentrate and recover a nucleic acid in a short time with high efficiency.

Broemeling et al in "An Instrument for Automated Purification of Nucleic Acids from Contaminated Forensic Samples", Journal of the Association for Laboratory Automation, Elsevier, vol. 13, no. 1, pp 40-38, January 2008 discloses a device which employs a rotating electric field pattern to concentrate DNA at the centre of an electrophoresis gel.

WO 02/42500 discloses a method and apparatus for electrophoretic microspot concentration of biomolecules. The method involves using the charge nature of the low molecules to localise them in a microspot containing a dialysis membrane. A channel filled with an electrophoresis gel may be provided in association with the microspot to allow biomolecules concentrated at the microspot to electrophoresis along the channel.

### SUMMARY OF THE INVENTION

Aspects of the invention are set out in the appended claims.

According to an embodiment of the present technology, there is provided a nucleic acid concentration recovery cartridge including:
a substrate,
a channel with which the substrate is provided to permit introduction of a liquid therein,
a cathode and anode arranged at opposite ends of the channel, respectively, and
a high molecular gel arranged in the channel at a predetermined position thereof, wherein the high molecular gel is operable to block moving nucleic acid towards the anode.

In the nucleic acid concentration recovery cartridge, the high molecular gel may preferably contain anionic functional groups, and the high molecular gel may preferably be configured to have a recessed shape from a side of the cathode toward a side of the anode.

In the nucleic acid concentration recovery cartridge, at least a portion of the channel may preferably be formed with a gel-holding portion that holds the high molecular gel in place. The gel-holding portion may have been subjected to hydrophilization treatment at a position thereof where the gel-holding portion is in contact with the high molecular gel. The gel-holding portion may preferably be coated with silica at the position thereof where the gel-holding portion is in contact with the high molecular gel. Further, the gel-holding portion may preferably have a predetermined shape such that the high molecular gel is fitted in the gel-holding portion and is held in place.

In addition, the channel may preferably have a greater volume between the high molecular gel and the cathode end compared with that between the anode end and the high molecular gel.

The cathode and anode may preferably have been formed by sputtering or vapor deposition. Further, the cathode and anode may preferably include gold or platinum.

According to another embodiment of the present invention, there is provided a nucleic acid concentration recovery method including filling, with a buffer, a channel provided with a high molecular gel arranged at a predetermined position thereof and also with a cathode and anode arranged at opposite ends of the channel, respectively, introducing a nucleic acid into the channel between the molecular gel and the cathode end, applying a voltage between the cathode and the anode to subject the nucleic acid to electrophoresis, and blocking the nucleic acid, which is moving toward the anode end, by the high molecular gel. According to this nucleic acid concentration recovery method, the nucleic acid can be concentrated in a vicinity of the high molecular gel by blocking the moving nucleic acid with the high molecular gel.

In the nucleic acid concentration recovery method, the high molecular gel may preferably contain anionic functional groups. Further, the high molecular gel may preferably be arranged in the channel to have a recessed shape in a direction of the electrophoresis of the nucleic acid.

Preferably, the nucleic acid concentration recovery method may further include applying a reverse voltage between the cathode and the anode after the electrophoresis.

According to a further embodiment of the present technology, there is also provided a fabrication process of the above-described nucleic acid concentration recovery cartridge, including gelling a monomer solution, which has been introduced in the channel with which the substrate is provided, into a predetermined shape through photopolymerization to form the high molecular gel.

In the fabrication process, the monomer solution may preferably contain anionic functional groups. The high molecular gel may preferably be gelled to have a recessed shape from a side of the cathode toward a side of the anode.

The nucleic acid concentration recovery cartridge according to the embodiment of the present disclosure is simple and convenient in operation, and can concentrate and recover a nucleic acid in a short time with high efficiency.

Embodiments of the disclosure provide a cartridge or the like that concentrates a nucleic acid by electrophoresis in a channel and recovers the same.

Further particular and preferred aspects of the present invention are set out in the accompanying independent and dependent claims. Features of the dependent claims may be combined with features of the independent claims as appropriate, and in combinations other than those explicitly set out in the claims.

### BRIEF DESCRIPTION OF THE DRAWINGS

The present invention will be described further, by way of example only, with reference to preferred embodiments thereof as illustrated in the accompanying drawings, in which:
FIGS. 1A to 1D are schematic views of a nucleic acid concentration recovery cartridge according to a first embodiment of the present technology and a procedure of a nucleic acid concentration recovery method making use of the cartridge;
FIGS. 2A and 2B are schematic views of modifications of the nucleic acid concentration recovery cartridge according to the first embodiment;
FIG. 3 is a schematic view of a further modification of the nucleic acid concentration recovery cartridge according to the first embodiment;
FIG. 4 is a schematic view of an even further modification of the nucleic acid concentration recovery cartridge according to the first embodiment;
FIG. 5 is a schematic view of a still further modification of the nucleic acid concentration recovery cartridge according to the first embodiment;
FIG. 6 is a schematic view of an even still further modification of the nucleic acid concentration recovery cartridge according to the first embodiment;
FIG. 7 is a schematic view of a yet further modification of the nucleic acid concentration recovery cartridge according to the first embodiment;
FIGS. 8A to 8D are schematic views of a nucleic acid concentration recovery cartridge according to a second embodiment of the present technology and a procedure of a nucleic acid concentration recovery method making use of the cartridge;
FIGS. 9A and 9B show photographs as substitutes for drawings, which illustrate observed fluorescence images of a channel in a vicinity of a high molecular gel (Example 2);
FIG. 10 is a diagram illustrating variations with time of fluorescence intensity at a gel interface after initiation of electrophoresis (Example 2);
FIG. 11 shows photographs as substitutes for drawings, which illustrate observed fluorescence images of a channel in a vicinity of a high molecular gel in a nucleic acid concentration recovery cartridge at varied pKa values of acids residual groups of which were anionic functional groups contained in the high molecular gel (Example 3); and
FIGS. 12A and 12B show photographs as substitutes for drawings, which illustrate observed fluorescence images of a channel in a vicinity of a high molecular gel (Example 4).

### DETAILED DESCRIPTION OF THE PREFERRED EMBODIMENTS

A description will hereinafter be made about preferred embodiments for practicing the present technology. It is to be noted that the embodiments to be described hereinafter are merely examples of representative examples of the present technology and shall by no means be construed to restrict the scope of the present technology. The description will be made in the following order:
1. Nucleic acid concentration recovery cartridge according to the first embodiment of the present technology and nucleic acid concentration recovery method making use of the cartridge
   (1) Nucleic acid concentration recovery cartridge
   (2) Nucleic acid concentration recovery method
2. Fabrication process of the nucleic acid concentration recovery cartridge according to the first embodiment of the present technology and modifications of the nucleic acid concentration recovery cartridge
   (1) Fabrication process of the nucleic acid concentration recovery cartridge
   (2) Modifications of the nucleic acid concentration recovery cartridge
   (3) Other modifications of the nucleic acid concentration recovery cartridge
3. Nucleic acid concentration recovery cartridge according to the second embodiment of the present technology and nucleic acid concentration recovery method making use of the cartridge
   (1) Nucleic acid concentration recovery cartridge
   (2) Nucleic acid concentration recovery method
4. Fabrication process of the nucleic acid concentration recovery cartridge according to the second embodiment of the present technology

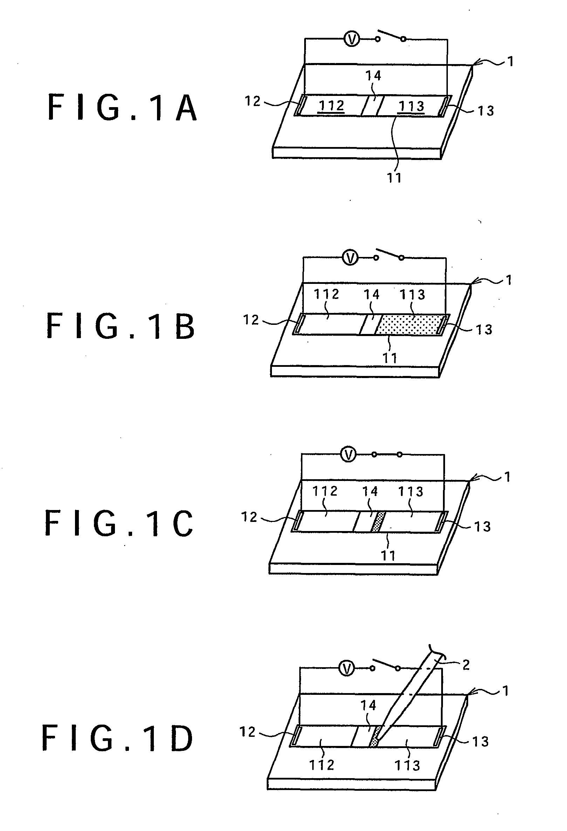

1. Nucleic acid concentration recovery cartridge according to the first embodiment of the present technology and nucleic acid concentration recovery method making use of the cartridge (1) Nucleic acid concentration recovery cartridge
Referring to FIGS. 1A to 1D, a description will be made of the construction of the nucleic acid concentration recovery cartridge according to the first embodiment of the present technology and the procedure of the nucleic acid concentration recovery method making use of the cartridge.

In FIG. 1A, the nucleic acid concentration recovery cartridge designated at numeral 1 has a substrate, a channel 11 formed on the substrate, and an anode 12 and cathode 13 arranged at opposite ends of the channel 11, respectively. The channel 11 is formed to permit introduction of a liquid therein, and a high molecular gel 14 is arranged between the anode 12 and the cathode 13 in the channel 11. Further, a power supply V is connected to the anode 12 and cathode 13 such that a voltage can be applied across or removed from the liquid introduced in the channel 11.

As the material of the substrate, glass or one of various plastics (polypropylene, polycarbonates, cycloolefin polymers, polydimethylsiloxane) may be employed.

As the anode 12 and cathode 13, those including gold (Au) or platinum (Pt) applied by sputtering or vapor deposition may be adopted suitably.

As the high molecular gel 14, a polyacrylamide may be adopted suitably. More preferably, a polyacrylamide containing anionic functional groups therein may be used, with the use of a polyacrylamide containing therein anionic functional groups, which are residual groups of an acid or a derivative thereof having an acid dissociation constant (pKa) of 1 to 5, being more preferred. It is to be noted that the term "acrylamide" as used in the present technology means an acrylamide or methacrylamide.

Examples of the anionic functional groups include, but are not specifically limited to, residual groups of carboxylic acids such as acetic acid, propionic acid and butyric acid; polybasic acids such as oxalic acid and phthalic acid; hydroxy acids such as citric acid, glycolic acid and lactic acid; unsaturated acids or unsaturated polybasic acids such as acrylic acid and methacrylic acid; and amino acids such as glycine, partial esters of phosphoric acid, partial esters of sulfuric acid, phosphonic acid, sulfonic acids, and so on.

Specific examples of the acid or derivative thereof include, as carboxylic acids, aliphatic monocarboxylic acids such as formic acid (pKa: 3.55), acetic acid (pKa: 4.56), propionic acid (pKa: 4.67), butyric acid (pKa: 4.63), pentenoic acid (pKa: 4.68), hexanoic acid (pKa: 4.63), heptanoic acid (pKa: 4.66), palmitic acid (pKa: 4.64) and stearic acid (pKa: 4.69); aliphatic or aromatic dicarboxylic acids such as succinic acid (pKa₁: 4.00, pKa₂: 5.24), glutaric acid (pKa₁: 4.13, pKa₂: 5.03), adipic acid (pKa₁: 4.26, pKa₂: 5.03), pimelic acid (pKa₁: 4.31, pKa₂: 5.08), suberic acid (pKa₁ : 4.35, pKa₂: 5.10), azelaic acid (pKa₁: 4.39, pKa₂: 5.12), malic acid (pKa₁: 3.24, pKa₂: 4.71) and terephthalic acid (pKa₁: 3.54, pKa₂: 4.46); unsaturated carboxylic acids such as crotonic acid (pKa: 4.69), acrylic acid (pKa: 4.26) and methacrylic acid (pKa: 4.66); substituted benzoic acids such as anisic acid (pKa: 4.09), m-aminobenzoic acid (pKa₁: 3.12, pKa₂: 4.74), m- or p-chlorobenzoic acid (pKa₁: 3.82, pKa₂: 3.99) and hydroxybenzoic acid (pKa₁: 4.08, pKa₂: 9.96); polycarboxylic acids such as citric acid (pKa₁: 2.87, pKa₂: 4.35, pKa₃: 5.69) and derivatives thereof.

As an acrylamide monomer containing an anionic functional group, an acrylamidoalkanesulfonic acid may be preferred. Examples of the sulfonic acid include sulfonic acids containing a polymerizable unsaturated group such as styrenesulfonic acid (pKa: -2.8), m-anilinesulfonic acid (pKa: 3.74), p-anilinesulfonic acid (pKa: 3.23), and 2-(meth)acrylamido-2-alkyl(carbon number: 1 to 4)propanesulfonic acid, more specifically 2-acrylamido-2-methylpropanesulfonic acid (pKa: -1.7).

The concentration (wt%) of the anionic functional groups in the polyacrylamide gel may preferably be 0 to 30%.
(2) Nucleic acid concentration recovery method

With reference to FIGS. 1B to 1D, a description will be made of a procedure of a nucleic acid concentration recovery method making use of the nucleic acid concentration recovery cartridge 1.

First, a sample that contains a nucleic acid therein is introduced into a region 113 between the high molecular gel 14 and the cathode 13 in the channel 11 (see, FIG. 1B). At this time, the cannel 11 has been filled with an electrophoresis buffer.

Next, a voltage is applied between the anode 12 and the cathode 13 to impress a voltage across the buffer, whereby the nucleic acid is subjected to electrophoresis. The negatively-charged nucleic acid is electrophoresed toward the anode 12 in the channel 11. On this occasion, the high molecular gel 14 blocks the movement of the nucleic acid and blocks the nucleic acid, so that the nucleic acid is concentrated at and in the vicinity of the interface of the high molecular gel 14 (see FIG. 1C). The electrophoresis time may be set as desired depending on the size of the channel 11, and can be, for example, 10 seconds to 10 minutes or so. It is to be noted that the term "interface" of the high molecular gel indicates a plane of contact between the high molecular gel 14 and the buffer filled on the side of the region 113.

Finally, the buffer in the vicinity of the high molecular gel 14 in the region 113 is taken up by a micropipette 2 or the like to recover the concentrated nucleic acid (see FIG. 1D).

At this time, the inclusion of anionic functional groups in the high molecular gel 14 makes it possible to prevent the nucleic acid from moving to the inside of the high molecular gel 14 owing to the electric repulsive force between the negatively-charged nucleic acid and the anionic functional groups. If the nucleic acid moves to the inside of the high molecular gel 14 or passes through the inside of the high molecular gel 14 and moves further to a region 112 on the side of the anode 12, the nucleic acid will be recovered in a smaller amount. The anionic functional groups may preferably be residual groups of an acid or a derivative thereof, the acid dissociation constant (pKa) of which is 1 to 5. As the acid or derivative thereof has a lower pKa and the functional groups have a greater negative charge, they are more effective for the hindrance of the movement of the nucleic acid into the high molecular gel 14 owing to the electrical repulsive force.

For the recovery of the nucleic acid in a greater amount, it is also effective to apply a reverse voltage between the anode 12 and the cathode 13 for a short time upon taking up the buffer in the vicinity of the high molecular gel 14 in the region 113. The application of the reverse voltage for the short time at the time of the recovery of the nucleic acid makes it possible to move the nucleic acid, which exists at the interface of the gel, and also the nucleic acid, which exists inside the gel and near the interface, to the vicinity of the high molecular gel 14 in the region 113 and to take up and recover the nucleic acid by the micropipette 2. The time, during which the reverse voltage is applied, may be set as desired depending on the size of the channel 11, and can be, for example, 1 to 10 seconds or so.

2. Fabrication process of the nucleic acid concentration recovery cartridge and modifications of the nucleic acid concentration recovery cartridge

### (1) Fabrication process of the nucleic acid concentration recovery cartridge

The nucleic acid concentration recovery cartridge 1 according to the first embodiment of the present technology can be fabricated by gelling a monomer solution, which has been introduced in the channel 11 formed on the substrate, into a predetermined shape through photopolymerization to form the high molecular gel 14.

The formation of the channel 11 on the substrate can be conducted, for example, by subjecting a glass-made substrate layer to wet etching or dry etching, by subjecting a plastic-made substrate layer to nanoimprint lithography, or by injection-molding a plastic-made substrate layer and then subjecting it to cutting work. It is to be noted that as the channel 11, one or more channels can be formed on the cartridge 1.

In the monomer solution, an acrylamide monomer may be suitably adopted. More preferably, an acrylamide monomer containing one or more anionic functional groups may be used, with the use of an acrylamide monomer containing one or more anionic functional groups, as a residual group or residual groups of an acid or a derivative thereof the acid dissociation constant (pKa) of which is 1 to 5 being still more preferred. Specifically, it is preferred to adopt an acrylamidoalkanesulfonic acid in the monomer solution.

The anode 12 and cathode 13 may preferably be formed by sputtering or vapor deposition of gold (Au) or platinum (Pt). In this respect, the cartridges according to the related technologies include those making use of platinum wires as electrodes. In this case, a need arises to arrange platinum wires in the nucleic acid concentration recovery cartridge whenever an experiment is performed. Moreover, in view of the high price of the platinum wires, the cartridge has not been provided as a disposable one. In the nucleic acid concentration recovery cartridge 1 according to this embodiment, on the other hand, the anode 12 and cathode 13 have been fabricated by sputtering or vapor deposition of gold (Au) or platinum (Pt) as mentioned above, and these electrodes have been formed beforehand in the cartridge 1. The cartridge can, therefore, be handled with ease. Such a nucleic acid concentration recovery cartridge 1 is disposable as opposed to the nucleic acid concentration recovery cartridges according to the related technologies. Accordingly, with the nucleic acid concentration recovery cartridge 1, it is possible to avoid contamination of a sample upon its concentration.

### (2) Modifications of the nucleic acid concentration recovery cartridge

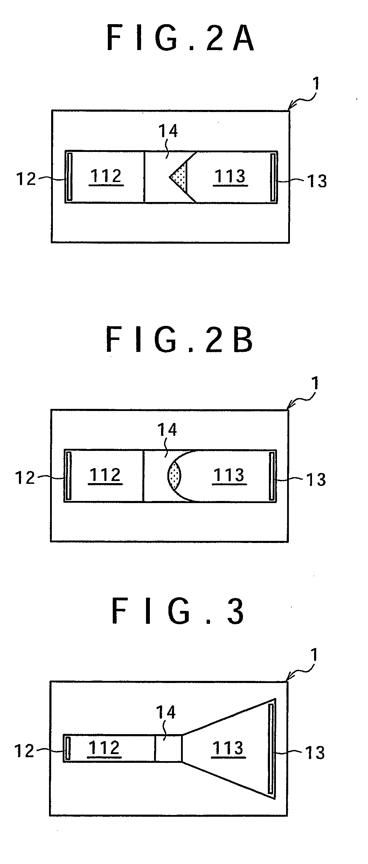

Referring to FIGS. 2A to 4, a description will be made of the constructions of modifications of the nucleic acid concentration recovery cartridge according to the first embodiment of the present technology.

In the nucleic acid concentration recovery cartridge according to the first embodiment of the present technology, the high molecular gel 14 may preferably be arranged in the channel 11 to have a recessed shape in the direction of electrophoresis of the nucleic acid. Described specifically, the high molecular gel 14 can be arranged in a configuration such that it is recessed in a triangular shape (FIG. 2A) or semicircular shape (FIG. 2B) from a side of the cathode 13 toward a side of the anode 12 as viewed from above.

When a voltage is applied between the anode 12 and the cathode 13 to electrophorese the negatively-charged nucleic acid toward the anode 12, the moving nucleic acid is blocked by the high molecular gel 14 and is concentrated in the vicinity of the high molecular gel 14 (see FIG. 1C). By arranging the high molecular gel 14 in the configuration that it is recessed from the side of the cathode 13 toward the side of the anode 12, the nucleic acid to be concentrated can be collected in the recessed portion at this time, thereby making it possible to increase the concentration rate of the nucleic acid and to efficiently recover the nucleic acid.

To configure the high molecular gel 14 in a shape recessed in the direction of electrophoresis of the nucleic acid, irradiation of light is conducted in conformity with the shape by exposure through a photomask, scanning of a laser beam, or a like method upon gelling through photopolymerization the monomer solution introduced in the channel 11.

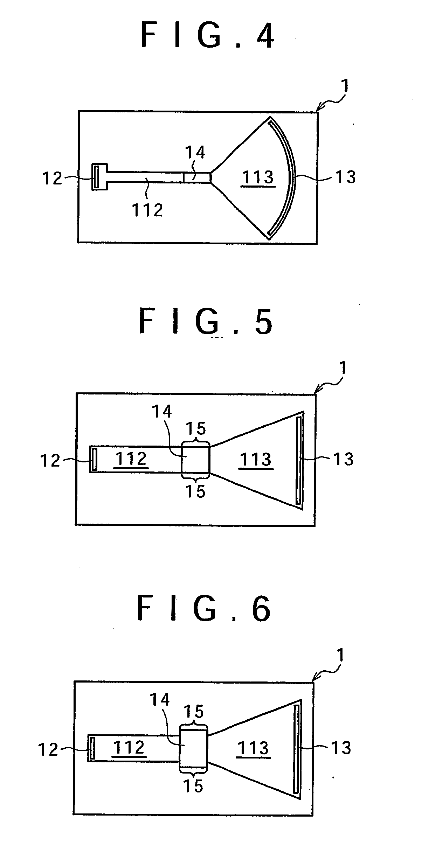

In the nucleic acid concentration recovery cartridge according to the first embodiment of the present technology, the channel volume of the region 113 (the region of the channel 11, which is located between the high molecular gel 14 and the cathode 13) into which a sample containing the nucleic acid is introduced may be formed greater compared with the channel volume of the region 112 between the anode end and the high molecular gel 14 (see FIG. 3). In this case, it is preferred to form the cathode side of the region 113 in the shape of a circular arc and also to arrange the cathode 13 in the shape of the same circular arc (see FIG. 4). When the nucleic acid concentration recovery cartridge is formed in the configurations shown in FIG. 4, the central angle of the circular arc may be set preferably at 0 to 30 degrees. Further, it is also suited to set the central angle at 30 to 60 degrees. Furthermore, it is also suited to set the central angle at 60 to 90 degrees. Moreover, it is also suited to set the central angle at 90 to 120 degrees.

The formation of the region 113 in a greater size makes it possible to introduce a sample solution in an increased volume, and therefore, to obtain the concentrated nucleic acid in a greater amount. In this modification, the formation of the cathode side of the region 113 and the cathode 13 in the shape of the circular arc makes it possible to form a more uniform electric field, and hence, to increase the efficiency of concentration of the nucleic acid.

### (3) Other modifications of the nucleic acid concentration recovery cartridge

Referring next to FIGS. 5 to 7, a description will be made of other modifications of the nucleic acid concentration recovery cartridge according to the first embodiment of the present technology. In the modifications to be described hereinafter with reference to FIGS. 5 to 7, the channels 11 are provided with gel holding portions 15, respectively, based by way of example on the nucleic acid concentration recovery cartridge 1 illustrated in FIG. 3, but the present technology shall not be limited to such exemplified modifications. Each of these gel holding portions 15 may also be arranged, for example, in the respective nucleic acid concentration recovery cartridges described with reference to FIGS. 1A to 1D, 2A, 2B and 3.

In the nucleic acid concentration recovery cartridge according to the first embodiment of the present technology, at least a portion of the channel 11 may preferably be formed with the gel-holding portion 15 that holds the high molecular gel 14 in place (see FIG. 5). The gel holding portion 15 may have been subjected to hydrophilization treatment, for example, at a position thereof where the gel-holding portion 15 is in contact with the high molecular gel 14. In this case, the gel-holding portion 15 may preferably be coated with silica at the position thereof where the gel-holding portion 15 is in contact with the high molecular gel 14.

When the gel-holding portion 15 has been subjected to hydrophilization treatment at the position thereof where the gel-holding portion 15 is in contact with the high molecular gel 14 as described above, the compatibility between the contact position and the high molecular gel 14 is improved, thereby making it possible to prevent the high molecular gel 14 from moving out of place upon conducting the concentration of the nucleic acid by electrophoresis. With the nucleic acid concentration recovery cartridge 1 according to this modification, the nucleic acid can hence be concentrated and recovered more stably owing to the arrangement of the gel-holding portion 15.

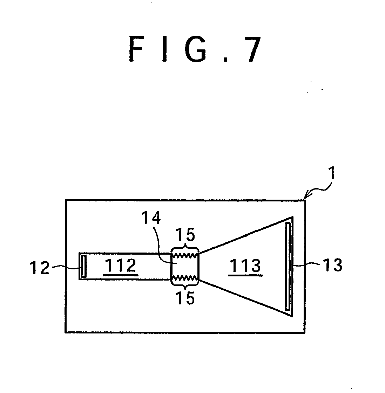

In the nucleic acid concentration recovery cartridge 1 according to each of the modifications of the first embodiment of the present technology, the gel-holding portion 15 may have a predetermined shape such that the high molecular gel 14 is fitted in the gel-holding portion 15. As depicted in FIG. 6, for example, the gel-holding portion 15 has a shape recessed in a direction perpendicular to the direction of electrophoresis of the nucleic acid such that an accommodation region for the high molecular gel 14 is enlarged, and therefore, can be maintained in fitting engagement with the high molecular gel 14. As illustrated by way of example in FIG. 7, on the other hand, the gel-holding portion 15 may have a shape that a plurality of fine nicks are formed, and therefore, can also be maintained in fitting engagement with the high molecular gel 14.

The nucleic acid concentration recovery cartridge 1 can also prevent the high molecular gel 14 from moving out of place upon conducting the concentration of the nucleic acid by electrophoresis when the high molecular gel 14 can be fitted in the gel-holding portion 15 as described above. With the nucleic acid concentration recovery cartridge 1, the nucleic acid can be concentrated and recovered more stably owing to the arrangement of the gel-holding portion 15.

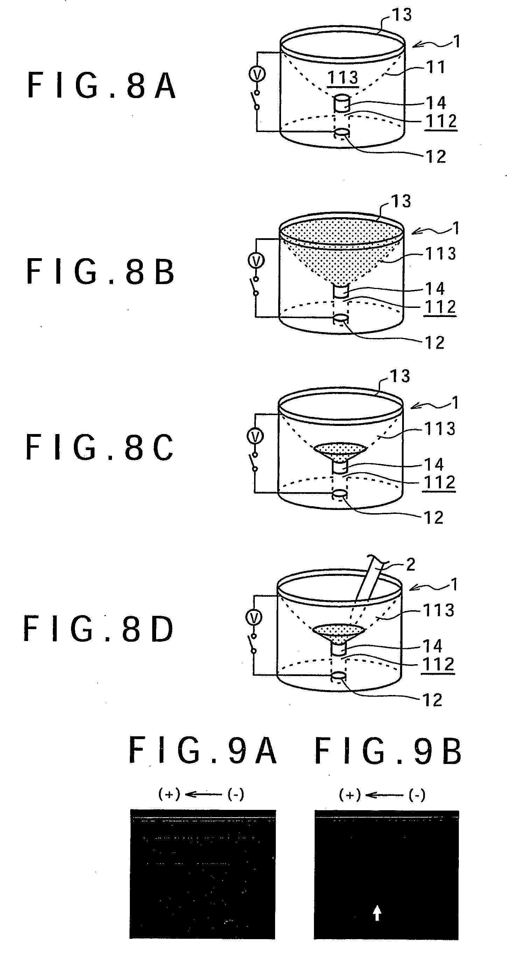

3. Nucleic acid concentration recovery cartridge according to the second embodiment of the present technology and nucleic acid concentration recovery method making use of the cartridge (1) Nucleic acid concentration recovery cartridge
Referring to FIGS. 8A to 8D, a description will be made of the construction of the nucleic acid concentration recovery cartridge according to the second embodiment of the present technology and the procedure of the nucleic acid concentration recovery method making use of the cartridge.

In FIG. 8A, the nucleic acid concentration recovery cartridge designated at numeral 1 has a substrate, a channel 11 formed through the substrate, and an anode 12 and cathode 13 arranged at opposite ends of the channel 11, respectively. The channel 11 is formed to permit introduction of a liquid therein, and a high molecular gel 14 is arranged between the anode 12 and the cathode 13 in the channel 11. Further, a power supply V is connected to the anode 12 and cathode 13 such that a voltage can be applied across or removed from the liquid introduced in the channel 11.

The channel 11 includes a region 113 into which the liquid is introduced. This region 113 will be described by taking a chamber of a conical shape as an example, but shall not be limited to such a shape. Various shapes such as, for example, a pyramid shape, prism shape and cylinder shape can also be adopted.

The external shape of the nucleic acid concentration recovery cartridge will be described by taking a cylinder shape as an example, but shall not be limited to such a shape. Various shapes such as, for example, a pyramid shape and prism shape can also be adopted.

As the high molecular gel 14, it is possible to choose a similar material and composition as in the above-mentioned nucleic acid concentration recovery cartridge according to the first embodiment.

Although not illustrated in FIGS. 8A to 8D, the gel-holding portions 15 arranged in the nucleic acid concentration recovery cartridge according to the first embodiment and described with reference to FIGS. 5 to 7 may each be arranged in the nucleic acid concentration recovery cartridge according to the second embodiment to prevent the high molecular gel 14 from moving out of place.

### (2) Nucleic acid concentration recovery method

With reference to FIGS. 8B to 8D, a description will be made of a procedure of a nucleic acid concentration recovery method making use of the nucleic acid concentration recovery cartridge 1 according to the second embodiment.

First, a sample that contains a nucleic acid therein is introduced in the region 113 between the high molecular gel 14 and the cathode 13 in the channel 11 (see, FIG. 8B). At this time, the channel 11 has been filled beforehand with an electrophoresis buffer.

Next, a voltage is applied between the anode 12 and the cathode 13 to impress it across the buffer, whereby the nucleic acid is subjected to electrophoresis. The negatively-charged nucleic acid is electrophoresed toward the anode 12 in the channel 11. On this occasion, the high molecular gel 14 blocks the movement of the nucleic acid and blocks the nucleic acid, so that the nucleic acid is concentrated at and in the vicinity of the interface of the high molecular gel 14 (see FIG. 8C). The electrophoresis time may be set as desired depending on the size of the channel 11, and can be, for example, 10 seconds to 10 minutes or so.

Finally, the buffer in the vicinity of the high molecular gel 14 in the region 113 is taken up by a micropipette 2 or the like to recover the concentrated nucleic acid (see FIG. 8D).

In the nucleic acid concentration recovery cartridge according to the second embodiment, the channel volume of the region 113 (the region of the channel 11, which is located between the high molecular gel 14 and the cathode 13) into which a sample containing the nucleic acid is introduced is formed very large compared with the channel volume of a region 112 between the anode end and the high molecular gel 14. The nucleic acid can, therefore, be concentrated and recovered efficiently in a large amount. Especially when, as illustrated in FIGS. 8A to 8D, the region 113 is configured to become progressively narrower in the direction in which the nucleic acid is electrophoresed, the nucleic acid can be concentrated and recovered more efficiently in a large amount.

Further, the inclusion of anionic functional groups in the high molecular gel 14 as in the nucleic acid concentration recovery cartridge according to the first embodiment makes it possible to prevent the nucleic acid from moving to the inside of the high molecular gel 14 owing to the electric repulsive force between the negatively-charged nucleic acid and the anionic functional groups. If the nucleic acid moves to the inside of the high molecular gel 14 or passes through the inside of the high molecular gel 14 and moves further to the region 112 on the side of the anode 12, the nucleic acid will be recovered in a smaller amount. The anionic functional groups may preferably be residual groups of an acid or a derivative thereof, the acid dissociation constant (pKa) of which is 1 to 5. As the acid or derivative thereof has a lower pKa and the functional groups have a greater negative charge, they are more effective for the hindrance of the movement of the nucleic acid into the high molecular gel 14 owing to the electrical repulsive force.

For the recovery of the nucleic acid in a greater amount, it is also effective to apply a reverse voltage between the anode 12 and the cathode 13 for a short time upon taking up the buffer in the vicinity of the high molecular gel 14 in the region 113. The application of the reverse voltage for the short time at the time of the recovery of the nucleic acid makes it possible to move the nucleic acid, which exists at the interface of the gel, and also the nucleic acid, which exists inside the gel and near the interface, to the vicinity of the high molecular gel 14 in the region 113 and to take up and recover the nucleic acid by the micropipette 2. The time, during which the reverse voltage is applied, may be set as desired depending on the size of the channel 11, and can be, for example, 1 to 10 seconds or so.

4. Fabrication process of the nucleic acid concentration recovery cartridge according to the second embodiment of the present technology

The nucleic acid concentration recovery cartridge 1 according to the second embodiment of the present technology can be fabricated by gelling a monomer solution, which has been introduced in the channel 11 formed through the substrate, into a predetermined shape through photopolymerization to form the high molecular gel 14.

In the monomer solution, an acrylamide monomer may be suitably adopted. More preferably, an acrylamide monomer containing one or more anionic functional groups may be used, with the use of an acrylamide monomer containing, as one or more anionic functional groups, a residual group or residual groups of an acid or a derivative thereof the acid dissociation constant (pKa) of which is 1 to 5 being still more preferred. Specifically, it is preferred to adopt an acrylamidoalkanesulfonic acid in the monomer solution.

As the anode 12 and cathode 13, those including gold (Au) or platinum (Pt) may be suitably adopted. In this case, the anode 12 and cathode 13 may be formed by sputtering or vapor deposition of gold or platinum. The nucleic acid concentration recovery cartridge 1 can be handled with ease because the anode 12 and cathode 13 have been formed beforehand in the cartridge by the sputtering or vacuum deposition of gold or platinum. Further, the nucleic acid concentration recovery cartridge 1 can be provided as a disposable one. With the nucleic acid concentration recovery cartridge 1, it is hence possible to avoid contamination of a sample.

Certain examples of representative embodiments of the description has been made about nucleic acid concentration recovery cartridge according to the present technology have been described above. The nucleic acid concentration recovery cartridge according to each embodiment was described by taking as an example one making use of the high molecular gel 14. In place of the high molecular gel 14, a commercially-available porous membrane such as a dialysis membrane, reverse osmosis membrane, semi-permeable membrane or ion exchange membrane or a commercially-available membrane of cellulose, polyacrylonitrile, a ceramic, zeolite, a polysulfone, a polyimide, palladium or the like may be used.

### Example 1

### 1. Fabrication of nucleic acid concentration recovery cartridge

On a PMMA substrate, a channel of 5 mm wide, 60 mm long and 2 mm deep was formed. At opposite ends of the channel an anode and cathode formed of platinum wires (diameter: 0.5 mm, length: 10 mm, product of The Nilaco Corporation) were arranged, respectively. The channel was filled with an acrylamide solution (Solution 2) prepared in accordance with Table 1 and Table 2. Using a confocal laser scanning microscope ("FV1000," trade name; manufactured by Olympus Corporation), photopolymerization of the acrylamide was conducted to form a high molecular gel of 5 mm wide and 3 mm long in the channel. Subsequently, the unpolymerized acrylamide solution was replaced to an electrophoresis buffer (1 x TBE).

**Table 1**

| | |
|---|---|
| Acrylamide | 1.67 g |
| | (16.7%) |
| Bisacrylamide | 0.53 g |
| | (5.30) |
| Acrylamidomethylpropanesulfonic acid (pKa 1) | 0.39 g |
| | (3.90) |
| x 10 TBE (pH 8.3) | 1 mL |
| Distilled water | 6.41 mL |
| Solution 1 | 10 mL |

**Table 2**

| | |
|---|---|
| Solution 1 | 1 mL |
| Riboflavin | 0.02 mL |
| TEMED | 0.02 mL |
| Solution 2 | Appox. 1 mL |

### Example 2

### 2. Concentration recovery of nucleic acid

Using the nucleic acid concentration recovery cartridge fabricated in Example 1, concentration of a nucleic acid was conducted. Between the high molecular gel and the cathode end in the channel, a sample (Cy3-modified oligonucleotide, 20 mer, 1 µg; 500 µl) was introduced. A voltage was applied between the cathode and the anode to conduct electrophoresis at 75 V and 1 mA. The moving sample was observed under the confocal laser scanning microscope.

Observed fluorescence images of the channel around the high molecular gel are shown in FIGS. 9A and 9B. The observed fluorescence image of FIG. 9A was taken before the initiation of electrophoresis, while that of FIG. 9B was taken three minutes after the initiation of the electrophoresis. In FIG. 9B, it is possible to confirm by fluorescence from Cy3 that the sample, which was moving from the side of the cathode toward the side of the anode under the electrophoresis, was blocked by the high molecular gel and was concentrated at and in the vicinity of the gel interface (see the arrow in FIG. 9B).

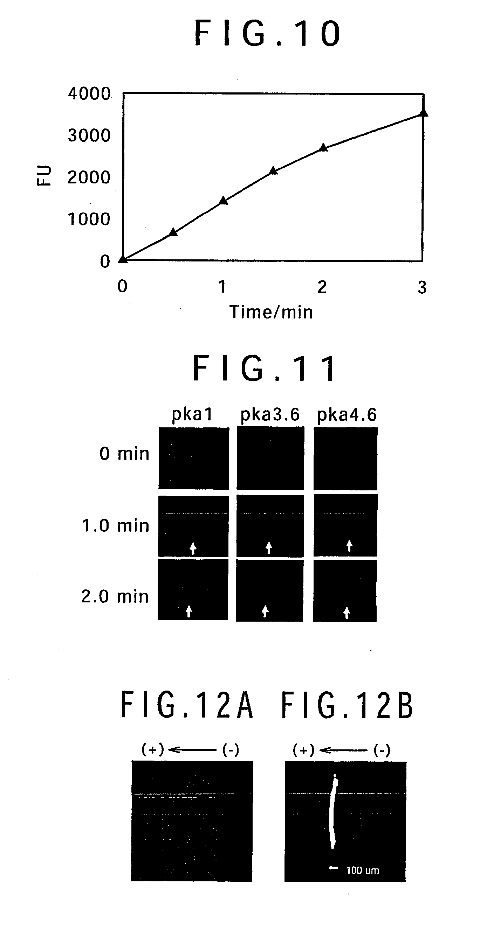

With reference to FIG. 10, a description will be made of variations with time of fluorescence intensity at the gel interface after the initiation of the electrophoresis in Example 2. It is understood that, after the initiation of electrophoresis, the intensity of fluorescence at the gel interface increased, and the sample, which was moving from the side of the cathode toward the side of the anode under the electrophoresis, was blocked by the high molecular gel and was concentrated at and in the vicinity of the gel interface. As a result of calculation of a concentration fold of the sample based on the fluorescence intensity, it was confirmed that a concentration fold as much as 44 fold was achieved three minutes after the initiation of the electrophoresis.

After completion of the electrophoresis, a reverse voltage (75 V, 1 mA) was applied for 10 seconds between the cathode and the anode to liberate the concentrated sample from the gel interface. By bringing a micropipette tip, the interior of which was controlled at a negative pressure, into contact with the gel interface, the concentrated sample was recovered as much as 10 µl.

### Example 3

### 3. Study on the acid dissociation constant (pKa) of high molecular gel

In this example, a relationship between the pKa of an acid, residual groups of which are anionic functional groups to be included in a high molecular gel and the efficiency of concentration of a nucleic acid was studied. Replacing acrylamidomethylpropanecarboxylic acid (pKa: 3.6) and acrylamidomethylcarboxylic acid (pKa: 4.6) to acrylamidomethylpropanesulfonic acid (pKa: 1.0) in the acrylamide solution (Solution 2) employed in Example 1, respectively, high molecular gels were formed, and nucleic acid concentration recovery cartridges were fabricated. Separately using the respective nucleic acid concentration recovery cartridges, the concentration of the nucleic acid was conducted by the method described in Example 2.

Observed fluorescence images of the channels of the respective nucleic acid concentration recovery cartridges around the high molecular gels are shown in FIG. 11. With the nucleic acid concentration recovery cartridge in which the high molecular gel including the residual groups of acrylamidomethylpropanecarboxylic acid (pKa: 3.6) or acrylamidomethylcarboxylic acid (pKa: 4.6) as anionic functional groups was formed, fluorescence was detected over a wide range, and a portion of the sample moved into the high molecular gel beyond the interface of the high molecular gel. With the nucleic acid concentration recovery cartridge in which the high molecular gel including the residual groups of acrylamidomethylpropanesulfonic acid (pKa: 1.0) as anionic functional groups was formed, on the other hand, fluorescence was detected in the form of a strip in a narrower range. It is, therefore, understood that the sample was concentrated with high efficiency at and in the vicinity of the gel interface.

### Example 4

### 4. Study on the capacity of channel

In this example, the concentration of a nucleic acid was conducted under similar conditions as in Example 1 except for the use of a nucleic acid concentration recovery cartridge of the shape shown in FIG. 4. The sector-shaped channel, which is located on the side of the cathode relative to the high molecular gel as a boundary, was formed in the shape of a sector having a radius of 40 mm and a central angle of 90 degrees, and had a depth of 2 mm. Therefore, the capacity of the channel was set at 2,512 (= [40 x 40 x 3.14 x 2]/4) µl. As the anode and cathode, those sputtered with gold (Au) were used. Now assuming that a region in which the nucleic acid is blocked by the high molecular gel 14 is 2 mm wide, 0.1 mm long and 2 mm deep, the nucleic acid can theoretically be concentrated up to 6,300 fold.

The results of actual electrophoresis of the sample are shown in FIG. 12A and 12B and Table 3. Observed fluorescence images of the channel around the high molecular gel are shown in FIGS. 12A and 12B. The observed fluorescence image of FIG. 12A was taken before the initiation of the electrophoresis, while that of FIG. 12B was taken 30 seconds after the initiation of the electrophoresis. Further, the measurement results of fluorescence intensity before the initiation of the electrophoresis and 30 seconds after the initiation of the electrophoresis are shown in Table 3.

**Table 3**

| Time (sec) | Fluorescence intensity | Concentration rate (fold) |
|---|---|---|
| 0 | 2.76 | - |
| 30 | 3,382 | 1,230 |

As appreciated from the foregoing, the use of the nucleic acid concentration recovery cartridge of the shape shown in FIG. 4 made it possible to achieve the concentration rate of 1,230 fold at 30 seconds after the initiation of electrophoresis. It has been demonstrated that compared with the results shown in Table 10, the nucleic acid concentration recovery cartridge of the shape shown in FIG. 4 can substantially improve the concentration fold at the 30 seconds after the initiation of electrophoresis.

The nucleic acid concentration recovery method according to the present technology is simple and convenient in operation, and can concentrate and recover a nucleic acid in a short time with high efficiency. Accordingly, the nucleic acid concentration recovery method can be applied to the concentration treatment of a nucleic acid for a nucleic acid amplification reaction such as the PCR (Polymerase Chain Reaction) method or the LAMP (Loop-Mediated Isothermal Amplification) method and can be used to detect the nucleic acid contained even in a trace amount or at a very low concentration in a sample.

The present disclosure contain subject matter related to that disclosed in Japanese Priority Patent Application JP 2011-017757 filed in the Japan Patent Office on January 31, 2011 and Japanese Priority Patent Application JP 2010-154692 filed in the Japan Patent Office on July 7, 2010.

## Claims

1. A nucleic acid concentration recovery cartridge (1) comprising:
a substrate,
a channel (11) with which the substrate is provided to permit introduction of a liquid therein,
a cathode (13) and anode (12) arranged at opposite ends of the channel, respectively, and
a molecular gel (14) arranged in the channel at a predetermined position thereof, wherein the molecular gel is a high molecular gel such that it is operable to block moving nucleic acid towards the anode.

2. The nucleic acid concentration recovery cartridge according to claim 1,
wherein the high molecular gel contains anionic functional groups.

3. The nucleic acid concentration recovery cartridge according to claim 2,
wherein the high molecular gel is configured to have a recessed shape from a side of the cathode toward a side of the anode.

4. The nucleic acid concentration recovery cartridge according to claim 1,
wherein at least a portion of the channel is formed with a gel-holding portion (15) that holds the high molecular gel in place.

5. The nucleic acid concentration recovery cartridge according to claim 4,
wherein the gel-holding portion has been subjected to hydrophilization treatment at a position thereof where the gel-holding portion is in contact with the high molecular gel.

6. The nucleic acid concentration recovery cartridge according to claim 5,
wherein the gel-holding portion is coated with silica at the position thereof where the gel-holding portion is in contact with the high molecular gel.

7. The nucleic acid concentration recovery cartridge according to claim 4,
wherein the gel-holding portion has a predetermined shape such that the high molecular gel is fitted in the gel-holding portion and is held in place.

8. The nucleic acid concentration recovery cartridge according to claim 1,
wherein the channel has a greater volume between the high molecular gel and the cathode end compared with that between the anode end and the high molecular gel.

9. The nucleic acid concentration recovery cartridge according to claim 1,
wherein the cathode and anode have been formed by sputtering or vapor deposition.

10. The nucleic acid concentration recovery cartridge according to claim 9,
wherein the cathode and anode include gold or platinum.

11. A nucleic acid concentration recovery method comprising:
filling, with a buffer, a channel (11) provided with a molecular gel (14) arranged at a predetermined position thereof and also with a cathode (13) and anode (12) arranged at opposite ends of the channel, wherein the molecular gel is a high molecular gel such that it is operable to block moving nucleic acid, respectively,
introducing a nucleic acid into the channel between the high molecular gel and the cathode end,
applying a voltage between the cathode and the anode to subject the nucleic acid to electrophoresis, and
blocking the nucleic acid, which is moving toward the anode end, by the high molecular gel.

12. The nucleic acid concentration recovery method according to claim 11,
wherein the high molecular gel is arranged in the channel to have a recessed shape in a direction of the electrophoresis of the nucleic acid.

13. The nucleic acid concentration recovery method according to claim 11, further comprising:
applying a reverse voltage between the cathode and the anode after the electrophoresis.

14. A fabrication process of a nucleic acid concentration recovery cartridge (1) including a substrate,
a channel (11) with which the substrate is provided to permit introduction of a liquid therein,
a cathode (13) and anode (12) arranged at opposite ends of the channel, respectively, and
a molecular gel (14) arranged in the channel at a predetermined position thereof, wherein the molecular gel is a high molecular gel such that it is operable to block moving nucleic acid towards the anode, comprising:
gelling a monomer solution, which has been introduced in the channel with which the substrate is provided, into a predetermined shape through photopolymerization to form the high molecular gel.

15. The fabrication process of the nucleic acid concentration recovery cartridge according to claim 14,
wherein the monomer solution contains anionic functional groups.

## Patentansprüche

1. Nukleinsäurekonzentrationskartusche (1), umfassend:
ein Substrat,
einen Kanal (11), mit dem das Substrat versehen ist, um das Einführen einer Flüssigkeit darin zu erlauben,
eine Kathode (13) bzw. eine Anode (12), die an gegenüberliegenden Enden des Kanals angeordnet sind, und
ein molekulares Gel (14), das in dem Kanal an einer vorbestimmten Position davon angeordnet ist, wobei das molekulare Gel ein hochmolekulares Gel ist, so dass es fähig ist, die Bewegung von Nukleinsäure in Richtung auf die Anode zu blockieren.

2. Nukleinsäurekonzentrationskartusche gemäß Anspruch 1,
wobei das hochmolekulare Gel anionische funktionelle Gruppen enthält.

3. Nukleinsäurekonzentrationskartusche gemäß Anspruch 2,
wobei das hochmolekulare Gel dafür gestaltet ist, eine vertiefte Form von einer Seite der Kathode in die Richtung auf eine Seite der Anode aufzuweisen.

4. Nukleinsäurekonzentrationskartusche gemäß Anspruch 1,
wobei wenigstens ein Abschnitt des Kanals mit einem Gel-haltenden Abschnitt (15) gebildet ist, der das hochmolekulare Gel an seinem Platz hält.

5. Nukleinsäurekonzentrationskartusche gemäß Anspruch 4,
wobei der Gel-haltende Abschnitt an einer Position davon, an der der Gel-haltende Abschnitt in Kontakt mit dem hochmolekularen Gel steht, einer Hydrophilisierungsbehandlung unterzogen worden ist.

6. Nukleinsäurekonzentrationskartusche gemäß Anspruch 5,
wobei der Gel-haltende Abschnitt an der Position davon, an der der Gel-haltende Abschnitt in Kontakt mit dem hochmolekularen Gel steht, mit Silica überzogen ist.

7. Nukleinsäurekonzentrationskartusche gemäß Anspruch 4,
wobei der Gel-haltende Abschnitt eine vorbestimmte Form aufweist, so dass das hochmolekulare Gel in den Gel-haltenden Abschnitt passt und an seinem Platz gehalten wird.

8. Nukleinsäurekonzentrationskartusche gemäß Anspruch 1,
wobei der Kanal ein größeres Volumen zwischen dem hochmolekularen Gel und dem Kathodenende aufweist als zwischen dem Anodenende und dem hochmolekularen Gel.

9. Nukleinsäurekonzentrationskartusche gemäß Anspruch 1,
wobei die Kathode und die Anode durch Sputtering oder Aufdampfen gebildet worden sind.

10. Nukleinsäurekonzentrationskartusche gemäß Anspruch 9,
wobei die Kathode und die Anode Gold oder Platin umfassen.

11. Nukleinsäurekonzentrationsverfahren, umfassend:
Füllen eines Kanals (11), der mit einem molekularen Gel (14), das an einer vorbestimmten Position davon angeordnet ist, und auch mit einer Kathode (13) bzw. einer Anode (12), die an gegenüberliegenden Enden des Kanals angeordnet sind, versehen ist, mit einem Puffer, wobei das molekulare Gel ein hochmolekulares Gel ist, so dass es fähig ist, die Bewegung von Nukleinsäure zu blockieren,
Einführen einer Nukleinsäure in den Kanal zwischen dem hochmolekularen Gel und dem Kathodenende,
Anlegen einer Spannung zwischen der Kathode und der Anode, um die Nukleinsäure an Elektrophorese zu unterwerfen, und
Blockieren der Nukleinsäure, die sich in Richtung auf das Anodenende bewegt, durch das hochmolekulare Gel.

12. Nukleinsäurekonzentrationsverfahren gemäß Anspruch 11,
wobei das hochmolekulare Gel in dem Kanal angeordnet ist, um eine vertiefte Form in einer Richtung der Elektrophorese der Nukleinsäure aufzuweisen.

13. Nukleinsäurekonzentrationsverfahren gemäß Anspruch 11, ferner umfassend:
Anlegen einer Umkehrspannung zwischen der Kathode und der Anode nach der Elektrophorese.

14. Herstellungsverfahren einer Nukleinsäurekonzentrationskartusche (1), umfassend ein Substrat,
einen Kanal (11), mit dem das Substrat versehen ist, um das Einführen einer Flüssigkeit darin zu erlauben,
eine Kathode (13) bzw. eine Anode (12), die an gegenüberliegenden Enden des Kanals angeordnet sind, und
ein molekulares Gel (14), das in dem Kanal an einer vorbestimmten Position davon angeordnet ist, wobei das molekulare Gel ein hochmolekulares Gel ist, so dass es fähig ist, die Bewegung von Nukleinsäure in Richtung auf die Anode zu blockieren, umfassend:
Gelieren einer Monomerlösung, die in den Kanal eingeführt worden ist, mit dem das Substrat versehen ist, in eine vorbestimmte Form durch Photopolymerisation, um das hochmolekulare Gel zu bilden.

15. Herstellungsverfahren der Nukleinsäurekonzentrationskartusche gemäß Anspruch 14,
wobei die Monomerlösung anionische funktionelle Gruppen enthält.

## Revendications

1. Cartouche de récupération de concentration d'acide nucléique (1) comprenant :
un substrat,
un canal (11) dont le substrat est doté pour y permettre l'introduction d'un liquide,
une cathode (13) et une anode (12) respectivement disposées à des extrémités opposées du canal, et
un gel moléculaire (14) placé dans le canal à une position prédéterminée de celui-ci, le gel moléculaire étant un gel à haut poids moléculaire de telle sorte qu'il est utilisable pour bloquer un acide nucléique en mouvement vers l'anode.

2. Cartouche de récupération de concentration d'acide nucléique selon la revendication 1, dans laquelle le gel à haut poids moléculaire contient des groupes fonctionnels anioniques.

3. Cartouche de récupération de concentration d'acide nucléique selon la revendication 2, dans laquelle le gel à haut poids moléculaire est configuré pour avoir une forme en creux depuis un côté de la cathode vers un côté de l'anode.

4. Cartouche de récupération de concentration d'acide nucléique selon la revendication 1, dans laquelle au moins une partie du canal est formée par une partie de maintien de gel (15) qui maintient le gel à haut poids moléculaire en place.

5. Cartouche de récupération de concentration d'acide nucléique selon la revendication 4, dans laquelle la partie de maintien de gel a été soumise à un traitement d'hydrophilisation à une position de celle-ci où la partie de maintien de gel est en contact avec le gel à haut poids moléculaire.

6. Cartouche de récupération de concentration d'acide nucléique selon la revendication 5, dans laquelle la partie de maintien de gel est recouverte de silice à la position de celle-ci où la partie de maintien de gel est en contact avec le gel à haut poids moléculaire.

7. Cartouche de récupération de concentration d'acide nucléique selon la revendication 4, dans laquelle la partie de maintien de gel a une forme prédéterminée telle que le gel à haut poids moléculaire est ajusté dans la partie de maintien de gel et est maintenu en place.

8. Cartouche de récupération de concentration d'acide nucléique selon la revendication 1, dans laquelle le canal a un volume plus important entre le gel à haut poids moléculaire et l'extrémité cathodique comparé à celui entre l'extrémité anodique et le gel à haut poids moléculaire.

9. Cartouche de récupération de concentration d'acide nucléique selon la revendication 1, dans laquelle la cathode et l'anode ont été formées par pulvérisation ou dépôt en phase vapeur.

10. Cartouche de récupération de concentration d'acide nucléique selon la revendication 9, dans laquelle la cathode et l'anode comprennent de l'or ou du platine.

11. Procédé de récupération de concentration d'acide nucléique comprenant :
le remplissage, avec un tampon, d'un canal (11) pourvu d'un gel moléculaire (14) placé à une position prédéterminée de celui-ci et également d'une cathode (13) et d'une anode (12) disposées à des extrémités opposées du canal, le gel moléculaire étant un gel à haut poids moléculaire de telle sorte qu'il est utilisable pour bloquer un acide nucléique en mouvement, respectivement,
l'introduction d'un acide nucléique dans le canal entre le gel à haut poids moléculaire et l'extrémité cathodique,
l'application d'une tension entre la cathode et l'anode pour soumettre l'acide nucléique à une électrophorèse, et
le blocage de l'acide nucléique, qui se déplace vers l'extrémité anodique, par le gel à haut poids moléculaire.

12. Procédé de récupération de concentration d'acide nucléique selon la revendication 11, dans lequel le gel à haut poids moléculaire est disposé dans le canal pour avoir une forme en creux dans la direction de l'électrophorèse de l'acide nucléique.

13. Procédé de récupération de concentration d'acide nucléique selon la revendication 11, comprenant en outre :
l'application d'une tension inverse entre la cathode et l'anode après l'électrophorèse.

14. Procédé de fabrication d'une cartouche de récupération de concentration d'acide nucléique (1) comprenant
un substrat,
un canal (11) dont le substrat est doté pour y permettre l'introduction d'un liquide,
une cathode (13) et une anode (12) respectivement disposées à des extrémités opposées du canal, et
un gel moléculaire (14) placé dans le canal à une position prédéterminée de celui-ci, le gel moléculaire étant un gel à haut poids moléculaire de telle sorte qu'il est utilisable pour bloquer un acide nucléique en mouvement vers l'anode, comprenant :
la gélification d'une solution de monomères, qui a été introduite dans le canal dont le substrat est doté, en une forme prédéterminée par photopolymérisation pour former le gel à haut poids moléculaire.

15. Procédé de fabrication de la cartouche de récupération de concentration d'acide nucléique selon la revendication 14, dans lequel la solution de monomères contient des groupes fonctionnels anioniques.
